# EUROPEAN PATENT SPECIFICATION

(11) **EP 0 871 457 B1**
(45) Date of publication and mention of the grant of the patent: **28.05.2003**
(21) Application number: 96912514.5
(22) Date of filing: 28.03.1996
(51) Int. Cl.: A61K 35/14, A61K 35/28, A61P 19/00

(54) **ISOLATED STROMAL CELLS AND METHODS OF USING THE SAME**
ISOLIERTE STROMAZELLEN UND METHODEN ZU DEREN VERWENDUNG
CELLULES DE STROMA ISOLEES ET LEUR METHODE D'UTILISATION

(30) Priority: 28.03.1995 US 412066; 13.11.1995 US 6627 P
(43) Date of publication of application: 21.10.1998
(60) Divisional application: 03075116.8
(73) Proprietor: THOMAS JEFFERSON UNIVERSITY, Philadelphia Pennsylvania 19107 (US)
(72) Inventor: PROCKOP, Darwin, J., Philadelphia, PA 19106 (US); PEREIRA, Ruth, F., Lansdowne, PA 19050 (US); LEEPER, Dennis, B., Wynnewood, PA 19096 (US); O'HARA, Michael, D., Wyncote, PA 19095 (US); KULKOSKY, Joseph, Philadelphia, PA 19111 (US); PHINNEY, Donald, Maple Glen, PA 19002 (US); LAPTEV, Alexey, Philadelphia, PA 19107 (US); CARO, Jose, Gladwyn, PA 19035 (US)
(74) Representative: Hallybone, Huw George
(86) International application number: US9604407
(87) International publication number: WO96030031

(56) References cited:
- EP-A- 0 381 490
- US-A- 4 904 259
- US-A- 5 334 640
- R.F. PEREIRA ET AL.: "CULTURED ADHERENT CELLS FROM MARROW CAN SERVE AS LONG-LASTING PRECURSOR CELLS FOR BONE, CARTILAGE, AND LUNG IN IRRADIATED MICE." PROCEEDINGS OF THE NATIONAL ACADEMY OF SCIENCES OF USA, vol. 92, May 1995 (1995-05), pages 4857-4861, XP002140403 WASHINGTON, US
- D.J. PROCKOP: "MARROW STROMAL CELLS AS STEM CELLS FOR NONHEMATOPOIETIC TISSUES." SCIENCE, vol. 276, 4 April 1997 (1997-04-04), pages 71-74, XP002140404 LANCASTER, PA, US
- THE NEW ENGLAND JOURNAL OF MEDICINE, 27 March 1980, Vol. 302, No. 13, COCCIA P.F. et al., "Successful Bone-Marrow Transplantation for Infantile Malignant Osteopetrosis", pages 702-707.
- ARTHRITIS AND RHEUMATISM, February 1993, Vol. 36, No. 2, NAKAGAWA T. et al., "Prevention of Autoimmune Inflammatory Polyarthritis in Male New Zealand Black/KN Mice by Transplantation of Bone Marrow Cells Plus Bone (Stromal Cells)", pages 263-268.
- BRITISH JOURNAL OF HAEMATOLOGY, 1983, Vol. 54, PIERSMA A.H. et al., "Transplantation of Bone Marrow Fibroblastoid Stromal Cells in Mice Via the Intravenous Route", pages 285-290.
- BIOMATERIALS, 1993, Vol. 14, No. 2, NIEDZWIEDZKI T. et al., "Bone Healing After Bone Marrow Stromal Cell Transplantation to the Bone Defect", pages 115-121.
- THE JOURNAL OF BONE AND JOINT SURGERY, April 1994, Vol. 76-A, No. 4, WAKITANI S. et al., "Mesenchymal Cell-Based Repair of Large, Full-Thickness Defects of Articular Cartilage", pages 579-592.
- BLOOD, August 1980, Vol. 56, No. 2, CASTRO-MALASPINA H. et al., "Characterization of Human Bone Marrow Fibroblast Colony-Forming Cells (CFU-F) and their Progeny", pages 289-301.

## Description

### FIELD OF THE INVENTION

The present invention relates to compositions comprising isolated stromal cells, and to using stromal cells in the preparation of medicaments for the treatment of individuals who have diseases associated with bone, cartilage or lung tissue and, diseases associated with genetic defects and/or diseases or conditions that can be treated by administering or delivering beneficial proteins.

### BACKGROUND OF THE INVENTION

In addition to hematopoietic stem cells, bone marrow contains "stromal cells" which are mesenchymal precursor cells (Friedenstein, A. J. *et al., Exp. Hemat.* **4:**267-274 (1976) that are characterized by their adherence properties when bone marrow cells are removed and put on to plastic dishes. Within about four hours, stromal cells adhere to the plastic and can thus be isolated by removing non-adhered cells form the dishes. These bone marrow cells that tightly adhere to plastic have been studied extensively (Castro-Malaspina, H. *et al., Blood* **56:**289-301 (1980); Piersma, A. H. *et al., Exp. Hematol* **13:**237-243 (1985); Simmons, P. J. and Torok-Storb, *B., Blood* **78**:55-62 (1991); Beresford, J. N. *et al., J. Cell. Sci.* **102:**341-351 (1992); Liesveld, J. L. *et al., Blood* **73**:1794-1800 (1989); Liesveld, J. L. *et al., Exp. Hematot* **19:**63-70 (1990); and Bennett, J. H. *et al., J. Call. Sci.* **99**:131-139 (1991)). As used herein, the term "adherent cells" is meant to refer to stromal cells and the term "non-adherent cells" is meant to refer to hematopoietic precursor cells.

Stromal cells are believed to participate in the creation of the microenvironment with the bone marrow *in vivo*. When isolated, stromal cells are initially quiescent but eventually begin dividing so that they can be cultured *in vitro.* Expanded numbers of stromal cells can be established and maintained. Stromal cells have been used to generate colonies of fibroblastic adipocytic and osteogenic cells when cultured under appropriate conditions. If the adherent cells are cultured in the presence of hydrocortisone or other selective conditions, populations enriched for hematopoietic precursors or osteogenic cells are obtained (Carter, R. F. *et al., Blood* **79:**356-364 (1992) and Bienzle, D. *et al.*, Proc. *Natl. Acad. Sci USA,* **91**:350-354 (1994)).

There are several examples of the use of stromal cells. European Patent EP 0,381,490 discloses gene therapy using stromal cells. In particular, a method of treating hemophilia is disclosed. Stromal cells have been used to produce fibrous tissue, bone or cartilage when implanted into selective tissues *in vivo* (Ohgushi, *H. et al., Acte. Orthop. Scand.* **60:**334-339 (1989); Nakahara, H. *et al. J. Orthop. Res.* **9**:465-476 (1991); Niedzwiedski, T. *et al., Biomaterials* **14**:115-121 (1993); and Wakitani, S. *et al., J. Bone & Surg.* **76A:**579-592 (1994)). In some reports, stromal cells were used to generate bone or cartilage in vivo when implanted subcutaneously with a porous ceramic (Ohgushi, H. *et al.* Acta. Orthop. Scand. **60:**334-339 (1989)), intraperitoneally in a diffusion chamber (Nakahara, H. *et al.* J. Orthop. Res. **9**:465-476 (1991)), percutaneously into a surgically induced bone defect (Niedzwiedski, T. *et al.* Biomaterials. **14**:115-121 (1993)), or transplanted within a collagen gel to repair a surgical defect in a joint cartilage (Wakitani, S. *et al.* J. Bone & Surg. **76A:**579-592(1994)). Piersma, A. H. *et al.* (*Brit. J. Hematol.* **54:**285-290 (1983)) disclose that after intravenous bone marrow transplantation, the fibroblast colony-forming cells, which make up the hemopoietic stroma, lodge and remain in the host bone marrow. Stewart *et al. (Blood* **81**:2566-2571 (1993)) recently observed that unusually large and repeated administrations of whole marrow cells produced long-term engraftment of hematopoietic precursors into mice that had not undergone marrow ablation. Also, Bienzle *et al.* (*Proc. Natl. Acad. Sci USA,* **91:**350-354 (1994)) successfully used long-term bone marrow cultures as donor cells to permanently populate hematopoietic cells in dogs without marrow ablation. In some reports, stromal cells were used either as cells that established a microenvironment for the culture of hematopoietic precursors (Anklesaria, *PNAS USA* **84**:7681-7685 (1987)) or as a source of an enriched population of hematopoietic stem cells (Kiefer, *Blood* **78(10):**2577-2582 (1991)).

### SUMMARY OF THE INVENTION

One aspect of the present invention relates to the use of stromal cells in the preparation of a medicament for treating patients who are suffering from a disease, disorder or condition characterized by a mutated, non-functioning or under-expressed gene which results in a defect in the patient's bones, cartilage or lungs. The stromal cells of this aspect of the invention have been obtained from a bone marrow sample from the patient or a matched syngeneic donor, the adherent cells are isolated from the sample, said adherent cells are to be transfected with a normal copy of said mutated, non-functioning or under-expressed gene that is operably linked to functional regulatory elements, and the transfected adherent cells are to be administered to the patient intravenously.

### BRIEF DESCRIPTION OF THE FIGURE

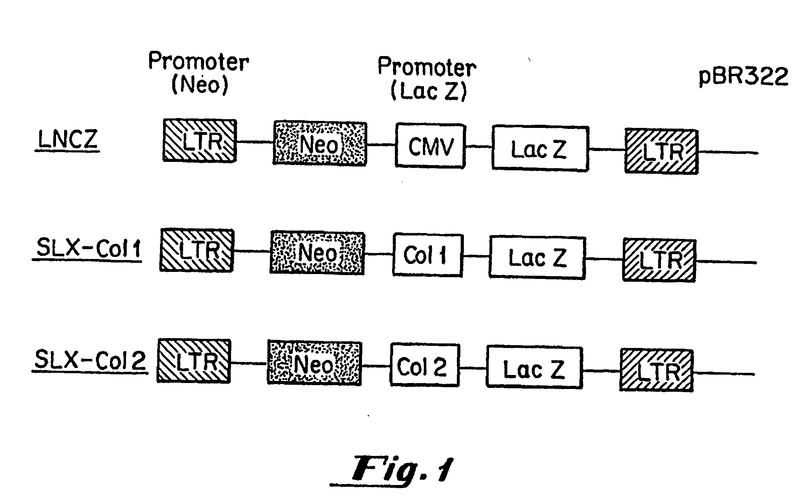

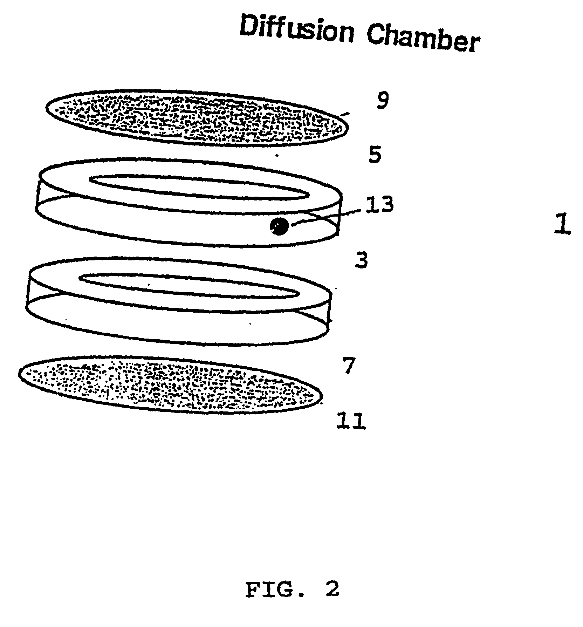

Figure 1 shows a schematic of the retroviral constructs pCMV-*lac Z,* pCOL1-*lac Z,* and pCOL2-*lac Z.* The cassettes of the gene constructs are: LTR-Neo-promoter-*Lac Z*-LTR.

### DETAILED DESCRIPTION OF THE INVENTION

As used herein, "stromal cells", "colony forming fibroblasts", "marrow stromal cells", "adherent cells" and "MSCs" are used interchangeably and meant to refer to the small fraction of cells in bone marrow which can serve as stem-cell-like precursors of osteocytes, chondrocytes, and adipocytes and which can be isolated from bone marrow by their ability to adhere to plastic dishes. Stromal cells may be derived from any animal. In some embodiments, stromal cells are derived from primates, preferably humans.

As used herein, "diseases, disorders and conditions characterized by a gene defect" is meant to refer to diseases, disorders and conditions in which defective genes and/or insufficient gene expression is causally linked to the disease or symptoms. Individuals who have any of several well known diseases, disorders and conditions characterized by a gene defect can be identified by those having ordinary skill in the art. Examples of diseases, disorders and conditions characterized by a gene defect include, but are not limited to, growth hormone deficiency, diabetes, adenine deaminase deficiency, hemophilia A and hemophilia B. The methods and means for diagnosing each such condition are well known.

As used herein, the term "disease, disorder or condition characterized by a bone, cartilage or lung defect" is meant to refer to diseases, disorders and conditions which are caused by a genetic mutation in a gene that is expressed by bone cells, cells which make cartilage or lung cells such that one of the effects of such a mutation is manifested by abnormal structure and/or function of the bone, cartilage and lungs respectively.

As used herein, "diseases, disorders and conditions characterized by a gene defect of a gene which encodes a secreted protein" is meant to refer to diseases, disorders and conditions characterized by a gene defect in which the gene that is defective or insufficiently expressed encodes a protein that is normally secreted.

As used herein, "diseases, disorders and conditions which can be treated with beneficial proteins" is meant to refer to diseases, disorders and conditions that can be treated or prevented by the presence of a protein which alleviates, reduces, prevents or causes to be alleviated, reduced or prevented, the causes and/or symptoms that characterize the disease, disorder or condition. Diseases, disorders and conditions which can be treated with proteins include diseases, disorders and conditions characterized by a gene defect.

As used herein, "beneficial protein" and "heterologous protein" are interchangeable and are meant to refer to 1) proteins which can compensate for the protein encoded by defective genes and/or insufficient gene expression that is causally linked to the disease or symptoms in diseases, disorders and conditions characterized by a gene defect and 2) proteins whose presence alleviates, reduces, prevents or causes to be alleviated, reduced or prevented, the causes and/or symptoms that characterize diseases, disorders and conditions which can be treated with beneficial proteins.

As used herein, "gene construct" is meant to refer to foreign recombinant nucleic acid molecules which include coding sequences that encode beneficial proteins operably linked to regulatory elements sufficient for expression of the coding sequence in stromal cells.

As used herein, "foreign recombinant nucleic acid molecules" is meant to refer to recombinant nucleic acid molecules which either are not present in stromal cells or are not expressed as proteins in sufficiently high levels in stromal cells until they are introduced into the cell by means such as, but not limited to, classical transfection (CaPO₄ or DEAE dextran), electroporation, microinjection, liposome-mediated transfer, chemical-mediated transfer, ligand mediated transfer or recombinant viral vector transfer.

As used herein, "heterologous gene" is meant to refer to the coding sequence of the gene construct.

As used herein, the terms "exogenous genetic material" and "exogenous gene" are used interchangeably and meant to refer to genomic DNA, cDNA, synthetic DNA and RNA, mRNA and antisense DNA and RNA which is introduced into the stromal cell. ' The exogenous genetic material may be heterologous or an additional copy or copies of genetic material normally found in the individual or animal. When cells are used as a component of a pharmaceutical composition for treating human diseases, conditions or disorders, the exogenous genetic material that is used to transform the cells may encode proteins selected as therapeutics used to treat the individual and/or to make the cells more amenable to transplantation.

As used herein, "transfected stromal cells" is meant to refer to stromal cells to which a gene construct has been provided through any technology used to introduce foreign nucleic acid molecules into cells such as, but not limited to, classical transfection (CaPO₄ or DEAE dextran), electroporation, microinjection, liposome-mediated transfer, chemical-mediated transfer, ligand mediated transfer or recombinant viral vector transfer.

Some aspects of the present invention arise from the discovery that some stromal cells which are introduced into a patient develop into bone, cartilage and lung while others remain precursors cells which throw off daughter cells that develop into bone, cartilage and lung. This discovery allows for the successful treatment of individuals suffering from diseases, conditions and disorders associated with defects in bone, cartilage or lung cells by either providing such individuals with stromal cells from a normal, matched syngeneic donor or by isolating stromal cells from the patient, culturing them and genetically modifying them to correct whatever genetic defect is responsible for the diseases, conditions and disorders associated with defects in bone, cartilage or lung cells.

The discovery that isolated, cultured stromal cells repopulate tissue, particularly bone, cartilage and lung tissue, when administered into the bloodstream of an individual makes them suited for use in the manufacture of a medicament for treating individuals suffering from diseases, conditions and disorders associated with defects in bone, cartilage or lung cells. The discovery that some isolated, cultured stromal cells, when administered into the blood stream of an individual, act as precursor cells which produce daughter cells that then mature into differentiated cells makes the invention particularly useful because it allows for the long term and continued presence of donated normal or genetically modified cells without the need for continuous readministration of cells.

Accordingly, stromal cells from a matched donor may be administered intravenously to individuals suffering from diseases associated with defective gene expression in bone, cartilage or lung cells, in order to replace the individual's bone, cartilage or lung cells that don't express or under express a normal gene and/or express a mutated gene. Stromal cells may also be transfected with heterologous genes in gene therapy protocols. According to such aspects of the invention, matched'donor stromal cells or stromal cells from an individual may be removed and genetically altered prior to reintroducing the cells into the individual. The cells may be genetically altered to introduce a gene whose expression has therapeutic effect on the individual. According to some aspects of the invention, stromal cells from an individual may be genetically altered to replace a defective gene and/or to introduce a gene whose expression has therapeutic effect on the individual.

In some aspects of the invention, cells that correctly express a normal gene may be used in the preparation of a medicament for treating individuals suffering from diseases and disorders that affect bone and that are characterized by a genetic defect wherein said cells supplement, augment and/or replace defective or deficient bone cells. The cells may be derived from stromal cells of a normal matched donor or stromal cells from the individual to be treated. If derived from the individual to be treated, the cells may be genetically modified to correct the defect. An example of a disease or disorder that affects bone and that is characterized by a genetic defect is osteogenesis imperfecta. Another example of a disease or disorder that affects bone and that is characterized by a genetic defect is osteoporosis. Osteoporosis is frequently regarded as a multifactorial disease to which environmental factors such as diet and exercise contribute. However, studies of disease in twins, large families and large populations demonstrate that many individuals develop the disease primarily because of a genetic defect (see Morrison *et al. Nature* **367**:284-287 (1994)). Stromal cells from a normal matched donor may be used in the preparation of a medicament for replacing the bone cells which have a mutated collagen gene in an individual who suffers from osteogenesis imperfecta. In such embodiments, the normal cells will compensate for the defective cells. In some embodiments, the normal cells may be prepared from the individual's own stromal cells, since cells with a mutated collagenous defect have a growth disadvantage compared to normal cells when grown in culture. Therefore, if stromal cells from an individual with osteogenesis imperfecta are grown as culture, they will gradually become enriched for normal cells. This embodiment will be particularly effective if the individual is a mosaic for the mutated collagen so that some of his or her cells contained the mutated collagen gene and others do not. In an alternative embodiment, stromal cells that have been isolated from an individual suffering from osteogenesis imperfecta are used, and into which a normal gene for collagen I has been inserted . The transfected cells may then be reintroduced into the individual. A few individuals suffering from osteoporosis also have mutations in one of the two genes for collagen I and the same embodiments will compensate for the defective cells. In most individuals with osteoporosis, the genes at fault are still unknown but are likely to be identified soon. In such individuals, normal cells will compensate for the defect. Also, when the genes at fault are identified and isolated, an alternative embodiment will be to use stromal cells that have been is isolated from the individual, and into which a normal copy or copies of the mutated gene has/have been inserted, in the preparation of a medicament for treating the individual, wherein said transfected cells are suitable for reintroduction into said individual.

In some aspects of the invention, cells that correctly express a normal gene may be used in the preparation of a medicament for the treatment of individuals suffering from diseases and disorders that affect cartilage and that are characterized by a genetic defect supplement, augment and/or replace defective cells . The cells may be derived from stromal cells of a normal matched donor or stromal cells from the individual to be treated. If derived from the individual to be treated, the cells may be genetically modified to correct the defect. An example of a disease or disorder that affects cartilage and that is characterized by a genetic defect is chondrodysplasia which cause severe dwarfism, severe problems with joints and related problems. Individuals suffering from chondrodysplasia may be administered stromal cells from a normal matched donor which replace the cells that produce cartilage in the individual which have a mutated. collagen gene. Here, the normal cells will compensate for the defective cells. Stromal cells may be isolated from an individual suffering from chondrodysplasia and a normal gene for collagen II may then be inserted into the isolated stromal cells. The transfected cells are then reintroduced into the individual. The embodiment with the collagen II gene will be useful for the 20% to 90% of individuals with various types of severe chondrodysplasia. The remaining individuals with chondrodysplasia have mutations in other collagen genes (collagen X and X1), in other genes (fibroblast growth factor receptor 3), and in still unidentified genes. In such individuals, normal cells will compensate for the defective cells. Also, in an alternative embodiment, stromal cells that have been isolated from the individual may be used, and into which a normal copy or copies of the mutated gene has/have been inserted. The transfected stromal cells may then be reintroduced to the individual. Another example of a disease or disorder that affects cartilage is osteoarthritis. Osteoarthritis is a heterogeneous disease both in terms of etiology and manifestations. Some individuals develop the degeneration of cartilage in joints that characterize osteoarthritis because of trauma or the late sequelae of infections. A few individuals develop osteoarthritis in multiple joints because of mutations in the gene for collagen II similar to the mutations in the gene that cause chondrodysplasia. Such individuals may or may not show signs of a mild' chondrodysplasia. The cause of osteoarthritis in other individuals is unknown, but studies in large families suggest that the disease is inherited and therefore caused by mutations in still unidentified genes. Therefore the same embodiments that will be useful to compensate for mutated genes in individuals with chondrodysplasia will also be useful for many individuals with osteoarthritis.

In some aspects of the invention, cells that correctly express a normal gene may be used in the preparation of a medicament for treating individuals suffering from diseases and disorders that affect the lungs and that are characterized by a genetic defect wherein said cells supplement, augment and/or replace defective cells. The cells may be derived from stromal cells of a normal matched donor or stromal cells from the individual to be treated. If derived from the individual to be treated, the cells may be genetically modified to correct the defect. An example of a disease or. disorder that affects the lungs and that is characterized by a genetic defect is cystic fibrosis. Another example of a disease or disorder that affects the lungs and that is characterized by a genetic defect is a deficiency of α1-antitrypsin. Individuals suffering from cystic fibrosis may be administered stromal cells from a normal matched donor which have a normal cystic fibrosis gene to replace or supplement the lung cells in the individual which have a mutated cystic fibrosis gene. Here, the normal cells will compensate for the defective cells. In an alternative embodiment, stromal cells may be isolated from an individual suffering from cystic fibrosis and a normal cystic fibrosis gene may then be inserted into the isolated stromal cells. The transfected cells can then be reintroduced into the individual.

In some aspects, expanded, rejuvinated stromal cells, which have been generated from isolated stromal cells, can be used in the preparation of a medicament for treating individuals suffering from diseases of the bone, cartilage and lungs. Some of the rejuvinated stromal cells will develop into normal bone, cartilage and lung cells. Normal stromal cells expand more quickly than the defective ones and the expanded rejuvinated populatation will reflect a greater proportion of normal cells. Table 1 describes media useful to culture expanded rejuvinated cultures of isolated stromal cells.

Gene constructs which comprise nucleotide sequences that encode heterologous proteins may be introduced into stromal cells. That is, the cells are genetically altered to introduce a gene whose expression has therapeutic effect on the individual. According to some aspects of the invention, stromal cells from an individual or from another individual may be genetically altered to replace a defective gene and/or to introduce a gene whose expression has therapeutic effect on the individual.

In some aspects of the invention, individuals suffering from genetic diseases and disorders may be treated by supplementing, augmenting and/or replacing defective or deficient genes by providing immunologically isolated stromal cells containing gene constructs that include normal, functioning copies of the deficient gene. This aspect of the invention relates to gene therapy in which the-individual is provided with genes for which they are deficient in presence and/or function. The genes provided in the cell therapeutic compensate for the defective gene of the individual. Such. genes preferably encode proteins that are secreted.

In all cases in which a gene construct is transfected into a stromal cell, the heterologous gene is operably linked to regulatory sequences required to achieve expression of the gene in the stromal cell. Such regulatory sequences include a promoter and a polyadenylation signal.

The gene construct is preferably provided as an expression vector which includes the coding sequence for a heterologous protein operably linked to essential regulatory sequences such that when the vector is transfected into the cell, the coding sequence will be expressed by the cell. The coding sequence is operably linked to the regulatory elements necessary for expression of that sequence in the cells. The nucleotide sequence that encodes the protein may be cDNA, genomic DNA, synthesized DNA or a hybrid thereof or an RNA molecule such as mRNA.

The gene construct includes the nucleotide sequence encoding the beneficial protein operably linked to the regulatory elements may remain present in the cell as a functioning cytoplasmic molecule, a functioning episomal molecule or it may integrate into the cell's chromosomal DNA. Exogenous genetic material may be introduced into cells where it remains as separate genetic material in the form of a plasmid. Alternatively, linear DNA which can integrate into the chromosome may be introduced into the cell. When introducing DNA into the cell, reagents which promote DNA integration into chromosomes may be added. DNA sequences which are useful to promote integration may also be included in the DNA molecule. Alternatively, RNA may be introduced into the cell.

The regulatory elements necessary for gene expression include: a promoter, an initiation codon, a stop codon, and a polyadenylation signal. It is necessary that these elements be operable in the stromal cells or in cells that arise from the stromal cells after infusion into an individual. Moreover, it is necessary that these elements be operably linked to the nucleotide sequence that encodes the protein such that the nucleotide sequence can be expressed in the stromal cells and thus the protein can be produced. Initiation codons and stop codon are generally considered to be part of a nucleotide sequence that encodes the protein. However, it is necessary that these elements are functional in the stromal cells or cells that arise from stromal cells. Similarly, promoters and polyadenylation signals used must be functional within the stromal cells or cells that arise from stromal cells. Examples of promoters useful to practice the present invention include but are not limited to promoters that are active in many cells such as the cytomegalic virus promoter, SV40 promoters and retroviral promoters. Other examples of promoters useful to practice the present invention include but are not limited to tissue-specific promoters, i.e. promoters that function in some tissues but not in others; also, promoters of genes normally expressed in stromal cells with or without specific or general enhancer sequences. In some embodiments, promoters are used which constitutively express genes in stromal cells with or without enhancer sequences. Enhancer sequences are provided in such embodiments when appropriate or desirable

According to some embodiments, desired genes for transfection into stromal cells are operably linked to the human procollagen I promoter, human procollagen II promoter, and the human procollagen III promoter. In some embodiments, the genes are linked to relatively short 5'-fragments from either the COL1A1 or COL2A1 gene which comprise the promoter together with the some of the 5' translated and/or untranslated sequences of the gene. In some embodiments, the gene to be transfected is operably linked to a sequence that contains a 1.9 kb *Sph*I-*Hind*III fragment from the 5'-end of the human COL1A1. The fragment contains from -476 bp to +1440 bp of COL1A1 gene and, therefore, includes the promoter (476 bp), exon 1 (222 bp) and most of the intron 1 (1223 bp of a total of 1453 bp). In some embodiments, the gene to be transfected is operably linked to a sequence that contains a fragment from the 5'-end of the human COL2A1. In some embodiments, the fragment contains -4.0 kb of the COL2A1 promoter and the complete COL2A1 gene the one or more exons and introns sequentially from exon 1 to exon 15 and intron 1 to intron 14. Some constructs may be designed as taught in co-pending U.S. Serial Number 08/184,260 filed January 18, 1994 entitled "Methods of targeting DNA insertion into genome".

Examples of polyadenylation signals useful to practice the present invention includes but is not limited to human collagen I polyadenylation signal, human collagen II polyadenylation signal, and SV40 polyadenylation signal.

In order for exogenous genetic material in an expression vector to be expressed, the regulatory elements must be operably linked to the nucleotide sequence that encodes the protein. In order to maximize protein production, regulatory sequences may be selected which are well suited for gene expression in the desired cells. Moreover, codons may be selected which are most efficiently transcribed in the cell. One having ordinary skill in the art can produce exogenous genetic material as expression vectors which are functional in the desired cells.

It is also contemplated that regulatory elements may be selected to provide tissue specific expression of the protein. Thus, for example, specific promoters may be provided such that the heterologous gene will only be expressed in tissue where the immunologically isolated stromal cells are implanted.

The heterologous protein preferably includes a signal sequence which directs the transport and secretion of the heterologous protein in the stromal cell. The signal sequences is generally processed and removed upon secretion of the mature protein from the cell.

In addition to providing cells with genetic material that either 1) corrects genetic defects in the cells, and/or 2) encodes proteins which are otherwise not present in sufficient quantities and/or functional condition so that the genetic material corrects genetic defects of the individual, genetic material may also be introduced into the stromal cells used in the present invention to provide a means for selectively terminating such cells should such termination become desirable. Such means for targeting cells for destruction may be introduced into stromal cells which are to be otherwise genetically modified as well as those to which no other exogenous genetic material is to be introduced.

According to the invention, isolated stromal cells are furnished with genetic material which renders them specifically susceptible to destruction. For example, the stromal cells may be provided with genes that encode a receptor that can be specifically targeted with a cytotoxic agent. An expressible form of a gene that can be used to induce selective cell death can introduced into the cells. In such a system, cells expressing the protein encoded by the gene are susceptible to targeted killing under specific conditions or in the presence or absence of specific agents. For example, an expressible form of a herpes virus thymidine kinase (herpes tk) gene can be introduced into the cells and used to induce selective cell death. When the exogenous genetic material that includes (herpes tk) gene is introduced into the individual, herpes tk will be produced. If it is desirable or necessary to kill the implanted cells, the drug gangcyclovir can be administered to the individual and that drug will cause the selective killing of any cell producing herpes tk. Thus, a system can be provided which allows for the selective destruction of implanted cells.

Stromal cells may be obtained after the removal bone marrow cells from a donor and placing the cells in a sterile container with a plastic surface or other appropriate surface that the cells come into contact with. The stromal cells will adhere to the plastic surface within 30 minutes to about 3 days. After at least 30 minutes, preferably about four hours, the non-adhered cells may be removed and discarded. The adhered cells are stromal cells which are initially non-dividing. After about 2-4 days however the cells begin to proliferate and can be cultured to increase their numbers using standard cell culture techniques.

According to preferred embodiments, stromal cells are cultured in medium supplemented with 2-20% fetal calf serum or serum-free medium with or without additional supplements.

Isolated stromal cells may be transfected using well known techniques readily available to those having ordinary skill in the art. Foreign genes may be introduced into stromal cells by standard methods for introducing gene constructs into cell which will express the proteins encoded by the genes. In some embodiments, cells are transfected by calcium phosphate precipitation transfection, DEAE dextran transfection, electroporation, microinjection, liposome-mediated transfer, chemical-mediated transfer, ligand mediated transfer or recombinant viral vector transfer.

In some embodiments, recombinant adenovirus vectors are used to introduce DNA with desired sequences into the stromal cell. In some embodiments, recombinant retrovirus vectors are used to introduce DNA with desired sequences into the stromal cell. In some embodiments, standard CaPO₄, DEAE dextran or lipid carrier mediated transfection techniques are employed to incorporate desired DNA into dividing cells. Standard antibiotic resistance selection techniques can be used to identify and select transfected cells. In some embodiments, DNA is introduced directly into cells by microinjection. Similarly, well known electroporation or particle bombardment techniques can be used to introduce foreign DNA into isolated stromal cells. A second gene is usually co-transfected or linked to the therapeutic gene. The second gene is frequently a selectable antibiotic-resistance gene. Transfected cells can be selected by growing the cells in an antibiotic that will kill cells that do not take up the selectable gene. In most cases where the two genes are unlinked and co-transfected, the cells that survive the antibiotic treatment have both genes in them and express both of them.

After isolating the stromal cells, the cells can be administered upon isolation or after they have been cultured. Isolated stromal cells administered upon isolation are administered within about one hour after isolation. Generally, stromal cells may be administration immediately upon isolation in situations in which the donor is large and the recipient is an infant. It is preferred that stromal cells are cultured prior to administrations. Isolated stromal cells can be cultured from 1 hour to over a year. In some preferred embodiments, the isolated stromal are cultured prior to administration for a period of time sufficient to allow them to convert from non-cycling to replicating cells. In some embodiments, the isolated stromal cells are cultured for 3-30 days, preferably 5-14 days, more preferably 7-10 days. In some embodiments, the isolated stromal cells are cultured for 4 weeks to a year, preferably 6 weeks to 10 months, more preferably 3-6 months.

If the cells are transfected, either 1) isolated, non-cycling stromal cells are first transfected and then administered as non-cycling cells, 2) isolated, non-cycling stromal cells are first transfected, then cultured for a period of time sufficient to convert from non-cycling to replicating cells and then administered, 3) isolated, non-cycling stromal cells are first cultured for a period of time sufficient to convert from non-cycling to replicating cells, then transfected, and then administered, or 4) isolated, non-cycling stromal cells are first cultured for a period of time sufficient to convert from non-cycling to replicating cells, then transfected, then cultured and then administered. In some embodiments, stromal cells are isolated, transfected and immediately administered. It is preferred that stromal cells are cultured prior to transfection and/or administrations. Isolated stromal cells can be cultured for 3-30 days, in some embodiments 5-14 days, in some embodiments 7-10 days prior to transfection. Transfected stromal cells can be cultured for 3-30 days, in some embodiments 5-14 days, in some embodiments 7-10 days prior to administration. Isolated stromal cells can be cultured for 3-30 days, in some embodiments 5-14 days, in some embodiments 7-10 days prior to transfection and upon transfection, additionally cultured for 3-30 days, in some embodiments 5-14 days, in some embodiments 7-10 days prior to administration. In some embodiments, the isolated stromal cells are cultured for 4 weeks to a year, in some embodiments 6 weeks to 10 months, in some embodiments 3-6 months prior to transfection. Transfected stromal cells can be cultured for 4 weeks to a year, in some embodiments 6 weeks to 10 months, in some embodiments 3-6 months prior to administration. In some embodiments, the isolated stromal cells are cultured for 4 weeks to a year, in some embodiments 6 weeks to 10 months, in some embodiments 3-6 months prior to transfection and upon transfection, further cultured for 4 weeks to a year, in some embodiments 6 weeks to 10 months, in some embodiments 3-6 months prior to administration.

Isolated stromal cells may be transfected using well known techniques readily available to those having ordinary skill in the art. In some embodiments, recombinant adenovirus vectors are used to introduce DNA with desired sequences into the stromal cell. In some embodiments, standard CaPO₄, DEAE dextran or lipid carrier mediated transfection techniques are employed to incorporate desired DNA into dividing cells. Standard antibiotic resistance selection techniques can be used to identify and select transfected cells. In some embodiments, DNA is introduced directly into cells by microinjection. Similarly, well known electroporation or particle bombardment techniques can be used to introduce foreign DNA into isolated stromal cells.

For administration of stromal cells, the isolated stromal cells are removed from culture dishes, washed with saline, centrifuged to a pellet and resuspended in a glucose solution which is infused into the patient. In some embodiments, bone marrow ablation is undertaken prior to infusion in order to make space in the bone for introduced cells. Bone marrow ablation may be accomplished by X-radiating the individual to be treated, administering drugs such as cyclophosphamide or by a combination of X-radiation and drug administration. In some embodiments, bone marrow ablation is produced by administration of radioisotopes known to kill metastatic bone cells such as, for example, radioactive strontium, ¹³⁵Samarium or ¹⁶⁶Holmium (see Applebaum, F.R. *et al.* 1992 *Blood* **80**(6):1608-1613).

If bone marrow ablation precedes administration of stromal cells, the administration of stromal cells must be accompanied by the administration of non-adherent cells which comprise blood cell precursors necessary for survival. Such non-adherent cells may be saved from the same sample used as starting materials in the isolation of stromal cells and stored or they can be derived from a different sample. In some preferred embodiments, the non-adherent cells are provided by the recipient/patient. Prior to procedures which generate bone marrow ablation, a sample of the patient/recipients bone marrow is obtained and stored. The entire sample may be used or the non-adherent cells may be isolated and used to administer in conjunction with isolated stromal cells. Non-adherent cells administered in conjunction with administration of stromal cells may be administered separately before or after stromal cell administration or may be mixed with isolated stromal cells prior to administration.

Bone marrow ablation is optional. For example partial but not complete bone marrow ablation may be produced prior to administration of stromal cells or stromal cells can be administered without any bone marrow ablation.

It is preferable that between 10⁷ and 10¹³ cells per 100 kg person are administered per infusion. In some embodiments, between about 1-5 X 10⁸ and 1-5 X 10¹² cells are infused intravenously per 100 kg person. In some embodiments, between about 1 X 10⁹ and 5 X 10¹¹ cells are infused intravenously per 100 kg person. In some embodiments, 4 X 10⁹ cells are infused per 100 kg person. In some embodiments, 2 X 10¹¹ cells are infused per 100 kg person.

In some embodiments, a single administration of cells is provided. In some embodiments, multiple administrations are provided. In some embodiments, multiple administrations are provided over the course of 3-7 consecutive days. In some embodiments, 3-7 administrations are provided over the course of 3-7 consecutive days. In some embodiments, 5 administrations are provided over the course of 5 consecutive days.

In some embodiments, a single administration of between 10⁷ and 10¹³ cells per 100 kg person is provided, In some embodiments, a single administration of between about 1-5 X 10⁸ and 1-5 X 10¹² cells per 100 kg person is provided. In some embodiments, a single administration of between about 1 X 10⁹ and 5 X 10¹¹ cells per 100 kg person is provided. In some embodiments, a single administration of 4 X 10⁹ cells per 100 kg person is provided. In some embodiments, a single administration of 2 X 10¹¹ cells per 100 kg person is provided.

In some embodiments, multiple administrations of between 10⁷ and 10¹³ cells per 100 kg person are provided, In some embodiments, multiple administrations of between about 1-5 X 10⁸ and 1-5 X 10¹² cells per 100 kg person are provided. In some embodiments, multiple administrations of between about 1 X 10⁹ and 5 X 10¹¹ cells per 100 kg person are provided over the course of 3-7 consecutive days. In some embodiments, multiple administrations of 4 X 10⁹ cells per 100 kg person are provided over the course of 3-7 consecutive days. In some embodiments, multiple administrations of 2 X 10¹¹ cells per 100 kg person are provided over the course of 3-7 consecutive days. In some embodiments, 5 administrations of 3-5 X 10⁹ cells are provided over the course of 5 consecutive days. In some embodiments, 5 administrations of 4 X 10⁹ cells are provided over the course of 5 consecutive days. In some embodiments, 5 administrations of 1-3 X 10¹¹ cells are provided over the course of 5 consecutive days. In some embodiments, 5 administrations of 2 X 10¹¹ cells are provided over the course of 5 consecutive days.

As stated above, the bone marrow sample for transplantation may be derived from a matched donor. Those having ordinary skill in the art can readily identify matched donors using standard techniques and criteria.

Bone marrow samples for transplantation may be obtained from matched donors by standard techniques. In some embodiments, bone marrow ablation is undertaken prior to infusion in order to make space in the bone for introduced cells. Bone marrow ablation may be accomplished by X-radiating the individual to be treated, administering drugs such as cyclophosphamide or by a combination of X-radiation and drug administration. In some embodiments, bone marrow ablation is produced by administration of radioisotopes known to kill metastatic bone cells such as, for example, radioactive strontium, ¹³⁵Samarium or ¹⁶⁶Holmium (see Applebaum, F.R. *et al.* 1992 *Blood* **80**(6):1608-1613).

Bone marrow ablation is optional. In some embodiments, partial but not complete bone marrow ablation is produced prior to bone marrow transplantation. In some embodiments, bone marrow is administered without any bone marrow ablation.

It is preferred that the number of cells used in bone marrow transplantation for treating bone and cartilage disease exceed that which is normally used for other treatments. Between 3 to 10 times the normal bone marrow dosage per 100 kg person are administered per infusion.

In some embodiments, a single administration of cells is provided. In some embodiments, multiple administrations are provided. In some embodiments, multiple administrations are provided over the course of 3-7 consecutive days. In some embodiments, 3-7 administrations are provided over the course of 3-7 consecutive days. In some embodiments, 5 administrations are provided over the course of 5 consecutive days.

In some embodiments, a single administration of between 10⁷ and 10¹³ cells per 100 kg person is provided, In some embodiments, a single administration of between about 1-5 X 10⁸ and 1-5 X 10¹² cells per 100 kg person is provided. In some embodiments, a single administration of between about 1 X 10⁹ and 5 X 10¹¹ cells per 100 kg person is provided. In some embodiments, a single administration of 4 X 10⁹ cells per 100 kg person is provided. In some embodiments, a single administration of 2 X 10¹¹ cells per 100 kg person is provided.

In some embodiments, multiple administrations of between 10⁷ and 10¹³ cells per 100 kg person are provided, In some embodiments, multiple administrations of between about 1-5 X 10⁸ and 1-5 X 10¹² cells per 100 kg person are provided. In some embodiments, multiple administrations of between about 1 X 10⁹ and 5 X 10¹¹ cells per 100 kg person are provided over the course of 3-7 consecutive days. In some embodiments, multiple administrations of 4 X 10⁹ cells per 100 kg person are provided over the course of 3-7 consecutive days. In some embodiments, multiple administrations of 2 X 10¹¹ cells per 100 kg person are provided over the course of 3-7 consecutive days. In some embodiments, 5 administrations of 3-5 X 10⁹ cells are provided over the course of 5 consecutive days. In some embodiments, 5 administrations of 4 X 10⁹ cells are provided over the course of 5 consecutive days. In some embodiments, 5 administrations of 1-3 X 10¹¹ cells are provided over the course of 5 consecutive days. In some embodiments, 5 administrations of 2 X 10¹¹ cells are provided over the course of 5 consecutive days.

### EXAMPLES

### Example 1

Cells from a transgenic mouse line that expresses a human mini-gene for collagen I in a tissue-specific manner were used to see whether precursor mesenchymal cells from marrow that are expanded in culture can serve as long-term precursors of bone and other connective tissues after intravenous infusion into irradiated mice. The marker gene consisted of an internally deleted mini-gene for the human proα1 (I) chain of procollagen I that caused synthesis of shortened proα1 (I) chains (Khillan, J. S. *et al., J. Biol. Chem.* **266**:23373-23379 (1991); Pereira, R. *et al., J. Clin. Invest.* **91:**709-716 (1983); and Sokolov, B. P. *et al., Biochemistry* **32:**9242-9249 (1993)). Cells expressing the gene were obtained from a line of transgenic mice in which the copy number of the human mini-gene relative to the endogenous mouse gene was about 100 to 1, and the steady-state levels of mRNA from the human mini-gene relative to mRNA from the endogenous mouse gene was about 0.5:1 in most tissues.

Donor cells from marrow partially enriched for mesenchymal precursors were prepared by standard protocols (Friedenstein, A. J. *et al., Exp. Hemat*. **4:**267-274 (1976); Castro-Mataspina, H. *et al., Blood* **56:**289-301 (1980); Piersma, A. H. *et al., Exp. Hematol* **13:**237-243 (1985); Simmons, P. J. and Torok-Storb, B., *Blood* **78:**55-62 (1991); Beresford, J. N. *et al., J. Cell. Sci.* **102**:341-351 (1992); Liesveld, J. L. *et al., Blood* **73:**1794-1800 (1989); Liesveld, J. L. *et al., Exp. Hematot* **19:**63-70 (1990); and Bennett, J. H. *et al., J. Call. Sci.* **99:**131-139 (1991)). Briefly, the ends of long bones from the transgenic mice were cut, and the marrow was extracted with a pressurized syringe filled with α-MEM (Sigma) containing 10% fetal bovine serum (Atlanta Biologicals). About 10⁷ nucleated cells were plated onto 175 cm² plastic culture flasks in 25 ml of α-MEM containing 10% fetal bovine serum. After 4 h, the non-adherent cells were discarded by replacing the medium. Foci containing two to four fibroblast-like cells appeared in 2 to 3 days, and the foci grew to near-confluent colonies in about 1 wk. The yield was about 10⁷ cells per flask after trypsin digestion. By phase-contrast microscopy, most of the cells were fibroblast-like, but a few macrophages and adipocytes were also seen.

About 10⁵ of the cultured adherent cells were mixed with 6 X 10⁵ non-adherent cells obtained by incubation of marrow from normal mice for 4 h on 175 cm² flasks under the same conditions used for the initial isolation of the adherent cells. The mixture of about 7 X 10⁵ cells in 0.2 to 0.4 ml of α-MEM and 10% fetal bovine serum was injected into the tail vein of each recipient mouse.

Eight-week old mice from the same inbred FVB/N line were prepared to receive the donor cells by irradiation with a ¹³⁷Cu irradiator (Atomic Energy of Canada, Ltd.). The unit had a dose rate of 116 cG/min with a parallel opposed beam configuration. Each animal received 9.0 Gy in two fractions with a 4 h interval (4.5 Gy + 4.5 Gy) (O'Hara, M.D. *et al., Exp. Hemat* **19:**878-881 (1991)). One to 2 h after the second radiation fraction, the mixture of marked adherent cells and normal non-adherent cells was injected intravenously. Control irradiated mice that .did not receive a cell infusion died after 10 to 13 days of marrow failure.

To follow the fate of the donor cells, two PCR assays for the human COL1A1 mini-gene and the mouse endogenous COL1A1 gene were developed. With a two-primer assay, the values for the ratio of the human to mouse gene were linear over a range of 10⁻⁴ to about 10⁺¹ and, therefore, of about 10-⁶ to 10-¹ donor cells per recipient cell. With the three-primer assay, the values were linear over a range of about 10⁻³ to 10⁺² and, therefore, about 10-⁵ to 1 donor cell per recipient cell.

Assays of irradiated mice after one day indicated only trace amounts of the donor cells in marrow, spleen, bone, lung or brain (Table 1). Slightly higher levels were seen at seven days. At 30 days and 150 days, progeny of the donor cells accounted for 2.0 to 12% of the cells in marrow, spleen, bone and lung (Table 1). At 150 days, they also accounted for 1.5 to 5.0% of the cells in xiphoid cartilage that was dissected free of any mineralized or fibrous tissue under a microscope. Although the mean values appeared to show. a decrease between 1 and 5 months, there was no statistically significant decrease in the combined values for marrow, spleen, bone and lung between these two time periods (Table 1). Assays of non-irradiated mice revealed only very low levels of the donor cells at the same time points (< 0.0001 to 0.05%). PCR *in situ* assay of tissue sections of lung demonstrated that progeny of the donor cells were evenly distributed in the parenchyma of both alveoli and bronchi.

To confirm that progeny of the donor cells were present in cartilage, chondrocytes were isolated from xiphoid and articular cartilage by digestion at 37°C overnight with 6.5 mg/ml bacterial collagenase (Sigma) in DMEM. PCR assays indicated that progeny of the donor cells accounted for 2.5% of the isolated chondrocytes.

To determine whether the donor cells became functional mesenchymal cells in the tissues they populated, tissues from the recipient mice were assayed by RT-PCR for expression of the human mini-gene for collagen I contained in the donor cells. In three mice assayed at 150 days, the mini-gene was expressed in bone, a tissue in which over half the protein synthesized in collagen I. The expression in bone was confirmed by a similar assay on bone cells isolated from femur and cultured for 1 wk. Expression of the mini-gene for collagen I was more variable in marrow, spleen and lung, tissues in which the rate of collagen I synthesis is less than in bone. As expected, the mini-gene was not expressed in cartilage, a tissue in which about half the protein is synthesized in collagen II but in which there is no synthesis of collagen I. The mini-gene for collagen I was also not expressed in cultures of chondrocytes from the recipient mice that contained the marker gene and that synthesize collagen II but not collagen I.

Earlier reports have shown that assays of the cells with cytochemical markers or mRNAs indicated that the cells synthesized collagen I, collagen III, fibronectin, alkaline phosphatase and osteopontin, but did not have features characteristic of macrophages, granulocytes, T lymphocytes, B lymphocytes or endothelial cells. The results here demonstrate that after intravenous injection into irradiated mice, the expanded cultures of adherent cells efficiently populate several connective tissues. The-results also demonstrate that the cells serve as true precursor cells for these tissues, since they expressed the marker gene for collagen I in a tissue-specific manner, and they were diffusely incorporated into the mesenchymal parenchyma of lung.

### Example 2

### Conditions for Isolation and Culture of MSCs.

Conditions for culture of MSCs so that they expand but retain the stem-cell-like phenotype have been studied. Table I shows that co-culture of MSCs with pieces of bone increased the number of cells obtained after 1 wk. At the same time, co-culturing with bone decreased the alkaline phosphatase (APase) levels in the cells, an observation suggesting that the cells did not differentiate into osteoblasts. Also, there was a decrease in the levels of tartrate-resistant acid phosphatase (TRAP), an observation suggesting that the cells did not differentiate into osteoclasts. Similar effects were observed with secondary cultures of the MSCs. Therefore, the results suggest that co-culturing with pieces of bone may provide improved conditions for expansion of MSCs. Also, the medium of cultured bone pieces may be an important source of cytokines and growth factors for expansion of MSCs in culture.

In related experiments, it has been found that secondary cultures of MSCs can be maintained for long periods of time. MSCs can be passed in culture for over 4 months by trypsinization and re-plating. The cells are remarkably stable in stationary phase cultures. In one experiment, stationary cultures remained viable for over 4 months with re-feeding about once per wk. In another experiment, the cells remained viable when, through an oversight, they were left in an incubator without re-feeding for 1 month.

### Stable Transfection of MSCs with a Retrovirus Vector.

To obtain virus for infection of MSCs, the LNCZ retroviral vector (Miller, A.D. and Rosman, G.J. (1989) *BioTechniques* 7, 980-990) was modified so that the promoter for cytomegalovirus (pCMV) drove expression of the lacZ gene (Fig. 1). The vector was stably transfected into an amphotropic murine packaging cell line (PA317). Constitutive virus producer clones were isolated by G418 selection, and supernatant from the clones was used for infection of MSCs. Primary cultures MSCs (3 days old) were infected for three successive days with fresh supernatant from the producer line with the highest titer. Staining of the cells 5 days later indicated that about 15-20% of the cells typically expressed the lacZ gene. Several cultures of the infected cells were placed under selection with G418 (0.4 4µg/ml active concentration) for 5 days. Most of the cells recovered continued to express the *lacZ* gene. Modifications of LNCZ were also constructed so that expression of the lacZ gene is driven by the promoter of the COL1 A1 gene and the promoter of the COL2 A1 gene (pCOL2Al). Expression of the lacZ gene was successfully obtained in primary cultures of MSCs with both constructs.

### Replacement of Bone Cells in Transgenic Mice with Normal MSCs.

MSCs from normal mice were infused into the transgenic mice that expressed high levels of the mutated COL1 A1 gene (Tables II and III). One month after the infusion of normal MSCs into the osteoimperfecta (OI) mice (Table II), progeny of the donor cells accounted for 10 to 45% of the bone cells in recipient mice that had been irradiated with a maximally tolerated dose of X-ray (700 centi-Gray or cGy). Similar values were obtained with mice irradiated with one-half of the maximally tolerated dose of X-ray (350 cGy). However, reducing the dose to one-quarter (175 cGy) reduced the values in four mice to 0%, 5%, 10% and 40%. Similar results were obtained when OI transgenic mice were infused with large numbers of whole marrow cells from which MSCs were not removed (Table III).

In five recipient mice in which the synthesis of proα1(l) chains was examined (Tables II and III), the replacement of the recipient's bone cells by normal donor MSCs was accompanied by an increase in the ratio of normal proα1(l) chains to mutated proα1 (l) chains in bone. Hence, the replacement by normal cells was accompanied by the expected changes at the protein level.

### Example 3

### Long-term expression of the human genes for hGH, factor IX or Ob in stably transfected MSCs.

MSCs are isolated from mice and cultured under the conditions described in Pereira, R.F., *et al.* (1995) *Proc. Natl. Acad. Sci. USA* 92, 4857-4861. MSCs are infected with retroviral vectors or transfected with naked DNA to obtain clones that express the genes for human growth hormone (hGH), the human obesity protein (Ob), or the gene for human factor IX. Because a lacz gene has been successfully introduced into mouse MSCs with a retroviral vector, variants of the same vector are used. At the same time, MSCs are stably transfected with electroporation (Andreason, G.L and Evans, G.A. (1988) *BioTechniques* 6, 650-660 and Toneguzzo, F., *et al.* (1986) *Mol. Call. Biol., 6,* 703-706), lipofectamine and nuclear injection (Mercer, W.E., *et al.* (1992) In: *Antisease Strategies, Ann. N. Y. Acad. Sci. Biol.* 660, 209-218) so that larger endogenous genes can be used. Further, some of the potential disadvantages of retroviruses are avoided using alternative introduction methodology.

Standard conditions for isolation and culture are: Whole marrow is obtained from the tibias and femurs of 6- to 10-wk old FVB/N mice by cutting the ends of the bones and extruding the marrow with a syringe that contains 1 to 2 ml of ice-cold αMEM and 10% fetal bovine serum (FBS). The pooled marrow cells are dispersed by gentle shaking and counted in an automatic counter (Coulter model ZM). From 5 x 10⁶ to 5 x 10⁷ nucleated cells in 25 *ml* of α-MEM and 10% FBS are plated onto 75-cm² culture flasks. After 4 h or 3 days, the non-adherent cells are removed by replacing the medium. The adherent cells are-expanded as primary cultures for 10 to 12 days with a re-feeding about every 4 days. The cells are recovered by digestion with 0.25% trypsin and 1 to 5 mM EDTA for 5 min at 37°C followed by gentle scraping. The cells are diluted with α-MEM with 10% FBS and replated at a density of from 3 X 10⁴ to 1 X 10⁵ cells per 9.5 cm² in 6-well plates. Under these conditions, the doubling time of the cells is 19 to 22 hours. The secondary cultures are re-fed about every 4 days, and passed by trypsinization and replating under the same conditions.

### Preparation of Gene Constructs.

The retrovirus vector LNCX is used As the parent construct. Convenient cloning sites in the construct are used to prepare the modified constructs pRSV-lacZ, pCMV-lacZ, pCOL1-/LacZ and pCOL2-lacZ (Fig. 1). The pCOL1 promoter is a 1.4 kb fragment that contains 476 bp of the promoter, the first exon and most of the first intron of the human COL1A1 gene. The promoter has been shown in transgenic mice to express a promoterless form of the COL2A1 gene in a highly tissue specific and developmental specific manner (Sokolov, B.P., *et al.* (1995) *J. Biol. Chem.* 270, 9622-9629). The COL2A1 promoter is a 1 kb fragment from the human COL2A1 gene (Ala-Kokko, L., *et al.* (1991) *J. Biol. Chem.* 266, 14175-14178) that confers tissue-specificity of expression (Bradham, D.M., *et al.* (1994) *J. Cell Physiol. 158,* 61-68). The lacZ gene is replaced with the hGH gene (Nichols Laboratories) ; the OB gene (Considine, R.V., *et al.* (1995) *J. Clin. Invest.* 95, 2986-2988,) or the human factor IX gene (Genetic Therapy, Inc.)

### Use of the Retrovirus Vector.

### Retrovirus Producer Cell Lines.

To establish producer cell lines, amphotrophic retrovirus packaging murine cells PA317 were used. The cells were transfected at 20% confluency in 100 mm dishes by the calcium phosphate precipitation procedure (Promega) using 15 µg of plasmid DNA that was linearized by digestion with *Sca*I that cuts in the pBR322 region of the retrovirus vector. One day post-transfection G418 (GIBCO/BRL) was added to the medium at an active concentration of *1 mg*/*ml*. Neomycin-resistant colonies appeared at 7 to 10 days of selection and were isolated by cloning with mechanical rings. The clones were expanded and individual clones were tested for the ability to express lacZ by direct staining of duplicate wells. The titer of the virus produced by the positive cells was assayed by single addition of 50 µl of medium to HT-1080 human tumor cells grown to 20% confluency in 6-well microliter plates with 3 ml medium per well and in the presence of 4 µg/ml of polybrene. The titer was assayed by determining the number of HT-1080 cells that stained positively for expression of the lacZ gene. Typically, the titer was 1 x 10⁵ to 1 x 10⁶.

### Retrovirus Infection of Mouse MSCs.

Primary cultures of mouse MSCs were prepared as described above. After 3 days, the non-adherent marrow cells were discarded and fresh medium added. The cells were then infected with the retrovirus in the presence of 4 µg/ml of polybrene by addition of 1/4 vol of fresh supernate medium from stably transfected producer cells that had the highest titer of virus production. The infection was repeated on two additional successive days. The cells were then either stained directly for *lacZ* expression or divided into larger dishes and placed under selection with 0.4 mg/ml of G418 (active concentration). About 15 to 20% of primary cultures were positive for lacZ and most of the cells that survived G418 selection were positive for lacZ.

### Lipofectamine Transfection.

Primary cultures of MSCs were grown for 10 days in (α-MEM containing 10% FBS). After trypsinization and light scraping, the cells were seeded in a 6-well plate at a density of 10⁵ cells per well. The cells were grown for 2 days, then washed 2 times with PBS and incubated with a DNA-lipofectamine complex. The DNA-lipofectamine complex was prepared as follows: 6 µl of lipofectamine (GIBCO/BRL) were mixed with 1 µg of LINCZ DNA in 200 µl of α-MEM, incubated at room temperature for 30 min, and added to one well of a 6-well plate containing MSCs in 800 µl α-MEM. After 6 h incubation at 37°C, the DNA-lipofectamine complex was replaced with 2 ml of α-MEM containing 10% FBS. The cells were stained for *lacZ* or placed under G418 selection after 18 h incubation in FBS-containing medium. Positive clones were obtained, but they grew slowly, apparently because the cell density was too low after the G418 selection. To circumvent this situation, three different strategies can be used: (a) cells are plated at higher densities; (b) co-culture cell culture inserts will be placed over surviving clones early in the selection process and place fresh MSCs or pieces of bone in the inserts (see Table 1) on a daily basis to provide the necessary cell factors to stimulate growth; (c) at the time that selection with G418 has killed many of the non-transfected calls, the cultures are reseeded with MSCs that have been infected with a variant of the retrovirus LNCX (Fig. 1) in which the lacZ gene is replaced with a selectable gene for thymidine kinase. Therefore, the MSCs stably transfected with retrovirus are used to provide the necessary cytokines, growth factors, and cell interactions required for the initial growth of the transfected MSCs during selection in G418. We can then remove the cells infected with the retrovirus by negative selection with gangcyclovir. Delivery methods

### Nuclear Injections.

Nuclear injections are highly efficient as a means of transfecting some cells. Cells were plated in 60-mm dishes containing a 22 x 22 mm coverslip marked with a circle to delineate the area for microinjection. Cells were incubated in medium containing 0.1% CS for 5 days to induce growth arrest before microinjection. Under these conditions between 8 and 15% of the cells incorporated [³H]thymidine during continuous labeling for 24 h between days 5 and 6. Microinjection was performed using a Zeiss Axiovert inverted-microscope equipped with an Eppendorf microinjector and micromanipulator using commercially purchased glass-capillary femtotips (Eppendorf). All cells within a delineated area of the coverslip (usually 150-200) were microinjected into the nucleus with DNA at concentrations ranging from 0.01-1 0 µg/µl in 10 mM Tris buffer (pH 7.6). The injected cells will be expanded and assayed as described above.

### Electroporation

MSCs are treated with 0.25% trypsin and 1 to 5 mM EDTA for 5 min at room temperature and then harvested by scraping. The cells are pelletted by centrifugation at 4,000 x g for 10 min, and then are washed twice by resuspending the pellet in ice cold PBS (pH 7.4). MSCs are resuspended at 2 x 10⁶ cells per 0.8 ml and aliquoted into an electroporation cuvette (0.4 cm gap). The cells are incubated 10 min on ice, DNA is added to the suspension (5 - 50 µg), and the cells are chilled for an additional 10 min. The cell suspension is then electroporated using a commercial instrument (BioRad Gene Pulser; model 1652076) at an empirically determined field strength which yields the greatest percentage of cells that retain the exogenously added DNA. To determine the appropriate field strength for MSCs, titrations have been performed ranging from 0.25 - 2.5 kv/cm. Electroporation efficiency was monitored by introducing a lacZ gene (LNCZ vector) and then staining cells 48 to 72 h after electroporation.

### Assays.

### hGH

Expression of the hGH gene is monitored by assaying medium from clones of calls grown in 6-well microliter plates with an enzyme linked immunoabsorbent assay with a commercially available kit (GIBCO/BRL). In this assay, 0.1 ml of 2 X diluent buffer is added per well of a microliter plate. After 5 min, 0.1 ml of test sample is added and the plate incubated at 37°C for 30 min. The wells are washed 5 times and 0.2 ml of primary antibody added per well. The samples are incubated at 37°C for 30 min, and washed 5 times. Then 0.2 ml of substrate buffer containing O-phenylenediamine substrate is added. Samples are incubated at room temperature for 30 min and the reaction stopped by addition of 0.1 ml of 2 N sulfuric acid. The absorbance of the sample is assayed at 490 nm.

### Ob Protein.

Cells are assayed for expression of the OB gene with a protein radioimmunoassay of cell medium. The primary antibody for human OB protein was raised in rabbits against recombinant protein synthesized in an E. *coli* expression system and purified to homogeneity. The human protein is highly homologous to the mouse and, therefore, anti-human antibodies should cross-react with the mouse protein. If they do not, the short mouse cDNA (619 nt) is expressed in *E*. *coli*, the protein is purified and antibodies are prepared. Alternatively, synthetic peptides with the mouse sequence are purchased and use these to prepare antibodies. For the assay, recombinant human Ob protein was radiolabeled with -¹²⁵Iodine by the Bolton-Hunter method followed by gel filtration purification using Sephadex G-25. The specific activity obtained was -30 µCi/µg. Samples of assay (0.2 ml) were preincubated with primary antiserum (1:2000 dilution) in phosphate buffered saline containing 0.1% Triton X-100 for 16 h at 4°C in a total Volume of 0.4 ml. ¹²⁵I-Ob protein (-30,000 cpm carried in 100 µl) was then added and the incubation continued for an additional 24 h. The bound Ob protein (12±1%; nonspecific binding 1.4±0.1%) was immunoprecipitated by addition of 0.1 ml sheep anti-rabbit IgG serum (Antibodies, Inc., Davis, CA), 0.1 ml normal rabbit serum (GIBCO/BRL, Gaithersburg, MD), and 0.1 ml of 10% polyethylene glycol. The tubes were centrifuges for 15 min (2200 rpm), and unbound label decanted and the pellet counted in a Packard 5000 gamma counter (Downers Grove, IL). The concentration of Ob protein in unknown samples was calculated using Rodbard's unweighted four parametric logistic model. The limit of detection of this assay is 0.39 ng/ml. The intra-assay variance is 11.6% at 12 ng/ml with an interassay variance of 20.8% at 13.1 ng/ml.

### Human Factor IX.

Expression of the gene for factor IX will be assayed with a commercially available ELISA (American Bioproducts Company) under conditions similar to those used for the hGH assay (above). As reported by Smith et al. (74), the standard curve ranged from 1-50 ng/ml⁻¹ and the limit of sensitivity was 1 ng/ml-¹. The assay did not cross-react with mouse factor IX.

### Example 4

### Sustained expression of the three genes at physiologically important levels by systemic infusion of stably transfected MSCs into mice.

Experiments with the OI transgenic mice (Tables II and III) have demonstrated that cultured MSCs can serve as stem-cell-like precursors of bone, cartilage and other mesenchymal tissues after systemic infusion. Therefore, MSCs expressing hGH, the Ob protein or factor IX are infused into irradiated and nonirradiated mice to evaluate sustained expression of the genes *in vivo.*

### Infusion of MSCs.

Initially, MSCs are infused into mice under conditions such as those described in Table II (3-week old mice: 300 or 700 Gray irradiation; intraperitoneal injection; 1 x 10⁶ MSCs; and 2 x 10⁸ whole marrow cells). In addition, intravenous infusion is compared to intraperitoneal; and lower levels of X-ray irradiation are employed. Also, the cells are infused into embryos by Cesarean section. In preliminary trials, 50 µl of 5 x 10⁴ES were injected into the amnion of seven 13-day embryos; 6 of 7 were delivered as viable pups. Therefore, intrauterine injection of MSCs is feasible.

### Growth Curves.

Effective *in vivo* expression of hGH should increase the growth rate of mice and expression of the Ob protein should induce starvation. Therefore, the weight and size of the treated mice and of control littermates are monitored.

### Assays for Gene Expression.

Blood is obtained from the retro-orbital plexus of mice at 1 wk, 1 month, 3 month, 5 month, 10 month, and 20 month after infusion of the MSCs. hGH and factor IX are assayed by ELISA, and the Ob protein is assayed with a radioimmune assays. In addition, if measurable increases in human factor IX are obtained with ELISA, the procedure described in Smith, T.A.G., *et al.* (1993) *Nature Genet*. 5 397-402, to assay biologically active human factor IX. In this procedure, human factor IX was first captured in a microtiter well with the monoclonal antibody, BGIX1, and then activated by factor Xla. The active factor IX, in combination with factor VIII, converted factor X to Xa. Factor Xa cleaved the chromogenic substrate, S2765, yielding a yellow product. BGIX1-coated microtiter plates and Factor VIII were purchased from Elcatech, Inc. (Winston-Salem, NC). Factor XIa was purchased from Enzyme Research Labs, Inc. (South Bend, IN). Factor X, phospholipid solution, S-2765, and the thrombin inhibitor, 1-2581, were purchased from Kabi Pharmacia Hepar, Inc. (Franklin, OH). Four buffers were prepared: A, 50 mM Tris, 150 mM NaCl, 1% BSA, pH 7.5; B, 150 mM Tris, 5 mM CaCl₂1 10 mg/ml⁻¹ gelatin, pH 7.6; C, 50 mM Tris, 1 0 mM CaCl₂ pH 7.5; D, 50 mM Tris, 15OmM NaCl, pH 8.4. The Factor VIII/X reaction mix was prepared fresh by mixing equal quantities of the following stocks: factor VIII, 5 U ml⁻¹ in buffer A; factor X, 1 U ml⁻¹ in buffers; 1-2581, 34 µg/ml⁻¹ in buffer A; CaCl₂, 25 mM in water; and phospholipid. Plasma samples were diluted in buffer A and *100 µl* were added to each microtiter well. The plate was incubated for 90 min at room temperature and then washed five times with buffer B. 100 µl of Factor Xla (2 µg/ml⁻¹ in buffer C) were added to each well. After 30 min at 37°C, 100 µl of S2765 (0.5 mM in buffer D) were added to each well and the plate was incubated for 10 min at room temperature before the reaction was stopped by adding acetic acid to a final concentration of 10%. Absorbances at 405 nm were determined with a Bio-Rad microplate reader. The standard curve, prepared with dilutions of human normal pooled plasma, was linear from 3-25 ng/ml⁻¹. The assay did not cross react with mouse factor IX. Factor IX, levels of 250 ng per ml or 5% of normal are generally considered therapeutic and 100 to 150 ng/ml are considered beneficial.

**Table I.**

| **Conditions for Growth of Primary and Secondary Cultures of MSCs.** | | | | |
|---|---|---|---|---|
| MSCs | Culture conditions | Cells per well X10⁵ | APase^{a} (mmol min/mg) | TRAP^{a} (mmol min/mg) |
| Primary | Standard^{a} | 2.0 | 426 | 144 |
| | Co-cultured^{b} | 6.41 | 22.3 | 102 |
| | Co-cultured^{c} | 6.94 | 42.0 | 105 |
| | (Matrigel) | | | |
| | | | | |
| Secondary^{d} | Standard | 1.33 | 2,052 | 75 |
| | Co-cultured | 7.40 | 362 | 60.6 |
| | Co-cultured | 5.08 | 506 | 59.2 |
| | (Matrigel) | | | |

| | | | | |
|---|---|---|---|---|
| ^{a}Whole marrow cells (20 x 10⁶) from 6-week old mice were cultured in individual 9.5 cm² wells in 2 ml of 10% FCS and α-MEM. Non-adherent cells were removed on day 3 and the incubation continued in fresh medium until day 7. APase and TRAP was assayed as described in reference 55. | | | | |
| ^{b}Co-cultured with pieces of bone (one-half femur and one-half tibia) in cell culture inserts (23 mm; 3 µm pore size; Becton Dickinson). | | | | |
| ^{c}Same as ^{b}, with inserts coated with Matrigel. | | | | |
| ^{d}Primary cultures on day 10 were detached with 0.25% trypsin and 1 mM EDTA for 5 min at 37°C followed by gentle scraping. Cells from one well (2 x 10⁵) were diluted 1:4 and cultured in 9.5 cm² wells for 7 days with changes of medium on day 3 and day 6. ^{e}APase (20,54) and TRAP (55) Activities were per mg total protein. | | | | |

**Table II.**

| **Experiments with (a) Transgenic Mice as Recipients; (b) Normal MSCs as Donor Cells; and (c) Decreasing X-Ray Dose**. | | | | | |
|---|---|---|---|---|---|
| Recipient mice | X-ray (cG) | Donor Cells^{a} | | Decrease Bone replacement at 1 month (%) | in mutated proα1 (l) chains |
| | | MSCs | Whole marrow | | |
| Transgenic | 700 | 0.7x10⁶(N)^{a} | 15x10⁶ (TG)^{ab} | 10 to 45% | 26 to 73% |
| (3 wk) | | | | (n=3) | (n=3) |
| | | | | | |
| Transgenic | 350 | 1.2x10⁶ (N) | 2x 10⁶ (TG) | 28 to 60% | |
| (3 wk) | | | | (n = 4) | |
| | | | | | |
| Transgenic | 175 | 1.2x10⁶ (N) | 2x 10⁶ (TG) | 0 to 40% | |
| (3 wk) | | | | (n = 4) | |

| | | | | | |
|---|---|---|---|---|---|
| ^{a}(N), normal; (TG), transgenic. ^{b}MSCs removed from whole marrow cells before infusion by incubation on plastic culture dish for 4 h at 37°C. | | | | | |

**Table III.**

| **Experiments with (a) Tranagenic Mice as Recipients; (b) 10-Fold Increase in Whole Marrow Cells as Donor Cells; and (c) Decreasing X-Ray Dose.** | | | | | |
|---|---|---|---|---|---|
| Recipient mice | X-ray (cG) | Donor Cells^{a} | | Bone replacement at 1 month (%) | Decrease in mutated proα1 (l) chains |
| | | MSCs | Whole marrow | | |
| Transgenic | 700 | | 5x10⁶(N)^{a} | 20 to 38% | |
| (3 wk) | | | | (n = 3) | |
| | | | | | |
| Transgenic | 350 | | 16x10⁶ (N) | 50 to 78% | 21 to 24% |
| (3 wk) | | | (n = 3) | (n = 3) | (n = 2) |
| | | | | | |
| Transgenic | 350 | | 5x10⁶ (N) | 22 to 45% | |
| (3 wk) | | | | (n = 4) | |

| | | | | | |
|---|---|---|---|---|---|
| ^{a} (N) whole marrow from normal mice without any treatment to remove MSCs. | | | | | |

## Claims

1. The use of stromal cells in the preparation of a medicament for treating a patient who is suffering from a disease, disorder or condition **characterised by** a mutated, non-functioning or under-expressed gene which results in a defect in the bone, cartilage or lungs of said patient, wherein:-
(i) said stromal cells have been obtained from the bone marrow of said patient;
(ii) said stromal cells are transfected with a normal copy of said gene which is operably linked to functional regulatory elements;
(iii) said stromal cells are suitable for intravenous infusion; and
(iv) therapeutic benefit is conferred at the site of said defect.

2. The use of claim 1, wherein said patient has undergone bone marrow ablation prior to administration of said stromal cells.

3. The use of claim 2, wherein said medicament further comprises haematopoeitic precursor cells from a bone marrow sample from a donor who is not suffering from a disease, disorder or condition **characterised by** a bone, cartilage or lung defect, and who is syngeneic with said patient.

4. The use of claim 2, wherein said stromal cells are to be administered free from haematopoeitic precursor cells.

5. The use of any preceding claim, wherein said stromal cells are transfected with a gene construct that comprises a herpes thymidine kinase gene, wherein said gene is operably linked to regulatory sequences and is expressed by said stromal cells.

6. The use of any preceding claim, wherein said bone defect is osteogenesis imperfecta or osteoporosis.

7. The use of any preceding claim, wherein said cartilage defect is chondrodysplasia or osteoarthritis.

8. The use of any preceding claim, wherein said lung defect is cystic fibrosis.

## Patentansprüche

1. Verwendung von Stromazellen zur Herstellung eines Medikaments für die Behandlung eines Patienten, der unter einer Erkrankung, Störung oder Verfassung leidet, **gekennzeichnet durch** ein mutiertes, nicht-funktionierendes oder unterexprimiertes Gen, was in einem Defekt im Knochen, Knorpel oder in den Lungen des Patienten resultiert, wobei
(i) die Stromazellen aus dem Knochenmark des Patienten erhalten worden sind,
(ii) die Stromazellen mit einer normalen Kopie des Gens, welches funktionsfähig mit funktionellen, regulatorischen Elementen verknüpft ist, transfiziert werden,
(iii) die Stromazellen für eine intravenöse Infusion geeignet sind und
(iv) der therapeutische Nutzen auf die Stelle des Defekts übertragen wird.

2. Verwendung nach Anspruch 1, wobei der Patient sich einer Knochenmarksentfemung vor der Verabreichung der Stromazellen unterzogen hat.

3. Verwendung nach Anspruch 2, wobei das Medikament weiter hämatopoetische Vorläuferzellen aus einer Knochenmarksprobe eines Donors umfaßt, welcher nicht an einer Erkrankung, Störung oder Verfassung leidet, **gekennzeichnet durch** einen Knochen-, Knorpel- oder Lungendefekt, und welcher syngenetisch mit dem Patienten ist.

4. Verwendung nach Anspruch 2, wobei die Stromazellen frei von hämatopoetischen Vorläuferzellen zu verabreichen sind.

5. Verwendung nach einem der vorhergehenden Ansprüche, wobei die Stromazellen mit einem Genkonstrukt transfiziert werden, welches ein Herpes-Thymidinkinasegen umfaßt, wobei das Gen funktionsfähig mit regulatorischen Sequenzen verknüpft ist und durch die Stromazellen exprimiert wird.

6. Verwendung nach einem der vorhergehenden Ansprüche, wobei der Knochendefekt Osteogenesis imperfecta oder Osteoporose ist.

7. Verwendung nach einem der vorhergehenden Ansprüche, wobei der Knorpeldefekt Chondrodysplasie oder Osteoarthritis ist.

8. Verwendung nach einem der vorhergehenden Ansprüche, wobei der Lungendefekt zystische Fibrose ist.

## Revendications

1. Utilisation de cellules de stroma dans la préparation d'un médicament pour traiter un patient qui souffre d'une maladie, d'un désordre ou d'une condition **caractérisé par** un gène sous-exprimé, non fonctionnant, muté, qui résulte d'un défaut dans les os, les cartilages ou les poumons dudit patient, dans laquelle :
(i) lesdites cellules de stroma ont été obtenues à partir de moelle d'os dudit patient ;
(ii) lesdites cellules de stroma sont transfectées avec une copie normale dudit gène qui est lié de façon opérationnelle aux éléments de régulation fonctionnels ;
(iii) lesdites cellules de stroma sont appropriées pour une injection intraveineuse ; et
(iv) un avantage thérapeutique est conféré au site dudit défaut.

2. Utilisation selon la revendication 1, dans laquelle ledit patient a subi une ablation de moelle osseuse avant l'administration desdites cellules de stroma.

3. Utilisation selon la revendication 2, dans laquelle ledit médicament comprend, en outre, des cellules de précurseurs hématopoéitiques d'un échantillon de moelle osseuse d'un donneur qui ne souffre pas d'une maladie, d'un désordre ou d'une condition **caractérisé par** un défaut des os, des cartilages ou des poumons et qui est congénital audit patient.

4. Utilisation selon la revendication 2, dans laquelle lesdites cellules de stroma sont administrées exemptes de cellules de précurseurs hématopoéitiques.

5. Utilisation selon l'une quelconque des revendications précédentes, dans laquelle lesdites cellules de stroma sont transfectées avec une construction de gène qui comprend un gène kinase thymidine herpès, où ledit gène est relié de façon opérationnelle à des séquences régulatrices et est exprimé par lesdites cellules de stroma.

6. Utilisation selon l'une quelconque des revendications précédentes, dans laquelle ledit défaut des os est l'ostéoporose ou la dysplasie périostale.

7. Utilisation selon l'une quelconque des revendications précédentes, dans laquelle ledit défaut des cartilages est la chondrodysplasie ou l'ostéoarthrite.

8. Utilisation selon l'une quelconque des revendications précédentes, dans laquelle ledit défaut des poumons est la fibrose cystique.
